# EUROPEAN PATENT APPLICATION

(11) **EP 1 325 682 A1**
(43) Date of publication of application: **09.07.2003**
(21) Application number: 01204839.3
(22) Date of filing: 11.12.2001
(51) Int. Cl.: A23L 1/30, A61K 35/78, A61P 19/00

(54) **Food or pet food composition containing plant extracts for maintenance of bone health and prevention or treatment of bone diseases**

(71) Applicant: SOCIETE DES PRODUITS NESTLE S.A., 1800 Vevey (CH)
(72) Inventor: Offord-Cavin, Elizabeth, 1041 Poliez-Pittet (CH); Federici, Ermanno, 1005 Lausanne (CH); Lemaure, Bernard, 37000 Tours (FR); Courtois, Didier, 37550 St. Avertin (FR)

(57) **Abstract**

The present invention relates to a composition for maintenance of bone health or prevention, alleviation and/or treatment of bone disorders. It also relates to the use of the composition in the manufacture of a nutritional product, a supplement or a medicament; and a method of promoting bone growth or for the maintenance of bone health which comprises administering an effective amount of the composition.

## Description

This invention relates to a human or pet composition for maintenance of bone health or prevention, alleviation and/or treatment of bone disorders. It also relates to the use of the composition in the manufacture of a nutritional product, a supplement or a medicament; and a method of promoting bone growth or for the maintenance of bone health which comprises administering an effective amount of the composition.

### Background of the Invention

Healthy bones require effective bone remodeling involving an equilibrium between bone formation and resorption. Most bone diseases are due to increased bone resorption , rendering its inhibition a primary therapeutic objective, therefore most pharmaceutical agents, developed to date, are anti-resorptive. For example, estrogens block production of cytokines that promote osteoclast generation and differentiation. SERMs (selective estrogen response modulator), are being developed which provide benefits for bone health while reducing the risk of adverse hormonal effects on breast or endometrial tissues. It is assumed that they work by a similar mechanism to estrogen in bone. Bisphosphonates (such as alendronate, risedronate etc) concentrate in bone and are, to date, the most effective inhibitors of bone resorption. They inhibit a critical enzyme pathway, required for osteoclast activity and survival. Calcitonin is a polypeptide hormone that inhibits bone resorption by blocking osteoclast activity. New targets include blocking of the TNF receptor/ligand family members and their signalling pathways, particularly of RANK/RANKL, inhibition of bone-specific metalloproteinases such as cathepsin K or inhibition of specific kinases.

Development of therapeutic agents stimulating bone formation has lagged behind that of resorption. Some chemical or pharmaceutical agents are known for promoting bone growth in human. For example, WO 9619246 describes a method for promoting bone growth in a human patient by (normally intermittent not continuous) administration of parathyroid hormone, PTH-related protein or an agonist for at least one month. In WO 9619501, a pancreatic-derived factor inhibits the resorption of bone and stimulates bone cells to proliferate and increases the formation of bone.

Although these chemicals and pharmaceutical compounds have been proved for the treatment of bone disorders, it would be of interest to provide a safe, efficient nutritional way to promote bone growth and prevent or alleviate the symptoms of bone/joint disorders in mammals.

### Summary of the Invention

Accordingly, in a first aspect, the present invention provides a composition intended for the prevention, alleviation and/or treatment of bone disorders or maintenance of bone health in mammals, which comprises as an active ingredient an effective amount at least one plant or plant extract selected from the group consisting of *Taraxacum* and *Amelanchier*.

Remarkably, it has now been found that some plants or plant extracts have a particular positive effect on bone formation and repair, on maintenance of bone health or prevention, alleviation and/or treatment of bone disorders.

The composition according to the invention can be used for the manufacture of a nutritional product, a supplement, a treat or a medicament intended for humans or pets.

Administering to a mammal a composition according to the present invention, results in an improved bone regeneration during fracture healing. It helps to inhibit bone resorption. It also helps to increase bone formation and bone mineral density during growth and optimize peak bone mass. Also, this composition helps to decrease bone loss, in particular bone loss associated with age in mammals. It reduces risk of osteoporosis. Furthermore it helps to build cartilage in mammals, to prevent osteoarthritis in pets or humans, which results in a better activity or mobility of the individual.

In another aspect, the invention relates to the use of a plant or plant extract as described above, for the preparation of a composition intended for the prevention, alleviation and/or treatment of bone disorders or maintenance of bone health in mammals.

It also relates to the use of a plant or plant extract as described above, for the preparation of a composition intended for inhibiting bone resorption.

It further relates to the use of a plant or plant extract as described above, for the preparation of a composition intended for improving activity and/or mobility of pets or humans.

In addition, the invention provides a method for the treatment, alleviation and/or prophylaxis of bone disorder or maintenance of bone health in mammals which comprises administering an effective amount of a composition as described above.

The invention further provides a method of increasing bone formation, bone mineral density during growth and optimize peak bone mass, treating or preventing osteoporosis, stimulating bone regenaration during fracture healing which comprises administering an effective amount of a composition as described above.

It further relates to a method for the treatment, alleviation and/or prophylaxis of osteoarthritis in pets or in humans, comprising the step of feeding an individual, a composition as described above.

It further provides a method decreasing bone loss, in particular bone loss associated with age in humans and pets, comprising the step of feeding an individual, a composition as described above.

### Detailed Description of the Invention

With respect to the first object of the present invention, the plant or plant extract is selected from the group consisting of *Taraxacum* and *Amelanchier*.

In a preferred embodiment, the plant or plant extract is *Taraxacum officinale* (common dandelion), *Taraxacum kok-saghyz* or *Amelanchier ovalis*, *Amelanchier alnifolia, Amelanchier laevis, Amelanchier arborea, Amelanchier asiatica*, for example.

The plant according to the invention may be from any part of the plant source, e.g. leaves, tubers, fruit or roots. In a most preferred embodiment, leaves or roots of *Taraxacum* species, or fruits of *Amelanchier species*, or a mixture thereof are used. The plant or plant extract may be in the form of a dried, lyophilized extract of leaves, roots and/or fruits depending on the source of plant, or fresh plant, or enriched fraction obtained by inorganic or organic solvant extraction process known in the art.

The plant or plant extract according to the invention may be used in the preparation of a food composition. The said composition may be in the form of a nutritionally balanced food or pet food, a dietary supplement, a treat or a pharmaceutical composition.

The plant or plant extract may be used alone or in association with other plants such as chicory, tea, cocoa, or with other bioactive molecule such as antioxidants, fatty acids, prebiotic fibers, glucosamine, chondroitin sulphate, for example.

In one embodiment, a food composition for human consumption is prepared. This composition may be a nutritional complete formula, a dairy product, a chilled or shelf stable beverage, soup, a dietary supplement, a meal replacement, and a nutritional bar or a confectionery.

Apart from the plant extract according to the invention, the nutritional formula may comprise a source of protein. Dietary proteins are preferably used as a source of protein. The dietary proteins may be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein, whey proteins and soy proteins are particularly preferred. The composition may also contain a source of carbohydrates and a source of fat.

If the nutritional formula includes a fat source, the fat source preferably provides about 5% to about 55% of the energy of the nutritional formula; for example about 20% to about 50% of the energy. The lipids making up the fat source may be any suitable fat or fat mixtures. Vegetable fats are particularly suitable; for example soy oil, palm oil, coconut oil, safflower oil, sunflower oil, corn oil, canola oil, lecithins, and the like. Animal fats such as milk fats may also be added if desired.

A source of carbohydrate may be added to the nutritional formula. It preferably provides about 40% to about 80% of the energy of the nutritional composition. Any suitable carbohydrates may be used, for example sucrose, lactose, glucose, fructose, corn syrup solids, and maltodextrins, and mixtures thereof. Dietary fibre may also be added if desired. If used, it preferably comprises up to about 5% of the energy of the nutritional formula. The dietary fibre may be from any suitable origin, including for example soy, pea, oat, pectin, guar gum, gum arabic, and fructooligosaccharides. Suitable vitamins and minerals may be included in the nutritional formula in an amount to meet the appropriate guidelines.

One or more food grade emulsifiers may be incorporated into the nutritional formula if desired; for example diacetyl tartaric acid esters of mono- and di- glycerides, lecithin and mono- and di-glycerides. Similarly suitable salts and stabilisers may be included. Vitamins and minerals may also be combined with the plant extract.

The nutritional formula is preferably enterally administrable; for example in the form of a powder, tablet, capsule, a liquid concentrate, solid product or a ready-to-drink beverage. If it is desired to produce a powdered nutritional formula, the homogenised mixture is transferred to a suitable drying apparatus such as a spray drier or freeze drier and converted to powder.

In another embodiment, a nutritional composition comprises a milk based cereal together with a prebiotic formulation. Preferably the milk based cereal is an infant cereal which acts as a carrier for the prebiotic formulation.

In another embodiment, a usual food product may be enriched with at least one plant or plant extract according to the present invention. For example, a fermented milk, a yoghurt, a fresh cheese, a renneted milk, article of confectionery, for example a sweet or sweetened beverage, a confectionery bar, breakfast cereal flakes or bars, drinks, milk powders, soy-based products, non-milk fermented products or nutritional supplements for clinical nutrition.

The amount of the plant or plant extract in the composition may vary according to the plant source and its utilization. In a preferred embodiment, an efficient daily dose amount is of at least about 1 mg, and more preferably from 1 mg to 200mg of the active molecule per day.

Also, the plant or plant extract according to the invention may be used in the preparation of a pet food composition. The said composition may be administered to the pet as a supplement to its normal diet or as a component of a nutritionally complete pet food, and more preferably in an hypocaloric pet food. It may also be a pharmaceutical composition.

Preferably, the pet food composition contains about 0.01 to 0.5 g of dry plants per gram of dry pet food for a 15 kg dog; and 0.001 to 0.1 g of dry plants per gram of wet pet food for a 15 kg dog.

The nutritionally complete pet food composition according to the invention may be in powdered, dried form, a treat or a wet, chilled or shelf stable pet food product. These pet foods may be produced by ways known in the art. Apart from the plant or plant extract, these pet foods may include any one or more of a starch source, a protein source and a lipid source. Suitable starch sources are, for example, grains and legumes such as corn, rice, wheat, barley, oats, soy, and mixtures of these. Suitable protein sources may be selected from any suitable animal or vegetable protein source; for example meat and meal, poultry meal, fish meal, soy protein concentrates, milk proteins, gluten, and the like. For elderly animals, it is preferred for the protein source to contain a high quality protein. Suitable lipid sources include meats, animal fats and vegetable fats.

The choice of the starch, protein and lipid sources will be largely determined by the nutritional needs of the animal, palatability considerations, and the type of product applied. For elderly pets, the pet food preferably contains proportionally less fat than pet foods for younger pets. Furthermore, the starch sources may include one or more of rice, barley, wheat and corn.

The pet food may optionally also contain a prebiotic, a probiotic micro-organism or another active agent, for example a long chain fatty acid. The amount of prebiotic in the pet food is preferably less than 10% by weight. For example, the prebiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight.

If a probiotic micro-organism is used, the pet food preferably contains about 10⁴ to about 10¹⁰ cells of the probiotic micro-organism per gram of the pet food; more preferably about 10⁶ to about 10⁸ cells of the probiotic micro-organism per gram. The pet food may contain about 0.5% to about 20% by weight of the mixture of the probiotic micro-organism; preferably about 1% to about 6% by weight; for example about 3% to about 6% by weight.

Suitable long chain fatty acids include linoleic acid, alpha-linolenic acid, gamma linolenic acid, eicosapentanoic acid, and docosahexanoic acid. Fish oils are a suitable source of eicosapentanoic acids and docosahexanoic acid. Borage oil, blackcurrent seed oil and evening primrose oil are suitable sources of gamma linoleic acid. Safflower oils, sunflower oils, corn oils and soybean oils are suitable sources of linoleic acid.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

For dried pet food a suitable process is extrusion cooking, although baking and other suitable processes may be used. When extrusion cooked, the dried pet food is usually provided in the form of a kibble. If a prebiotic is used, the prebiotic may be admixed with the other ingredients of the dried pet food prior to processing. A suitable process is described in European patent application No 0850569. If a probiotic micro-organism is used, the organism is preferably coated onto or filled into the dried pet food. A suitable process is described in European patent application No 0862863.

For wet food, the processes described in US patents 4,781,939 and 5,132,137 may be used to produce simulated meat products. Other procedures for producing chunk type products may also be used; for example cooking in a steam oven. Alternatively, loaf type products may be produced by emulsifying a suitable meat material to produce a meat emulsion, adding a suitable gelling agent, and heating the meat emulsion prior to filling into cans or other containers.

The amount of pet food to be consumed by the pet to obtain a beneficial effect will depend on the size of the pet, the type of pet, and age of the pet. However, an amount of the pet food to provide a daily amount of about 0.5 to 5 g of dry plants per kg of body weight, would usually be adequate for dogs and cats.

Administering to a human or animal, the food or pet food composition as described above, results in an improved bone regeneration during fracture healing. It helps to stimulate bone formation and bone mineral density during growth and optimize peak bone mass. In particular it may provide an optimal bone growth during childhood for humans or pets. This food composition helps to prevent bone loss, in particular bone loss associated with age in mammals or bone loss associated with long term hospitalization. It reduces risk of osteoporosis. Furthermore it helps to build cartilage in mammals, prevent osteoarthritis in humans and pets, which results in a better activity or mobility of the individual.

The following examples are given by way of illustration only and in no way should be construed as limiting the subject matter of the present application. All percentages are given by weight unless otherwise indicated. The examples are preceded by a brief description of the figure.

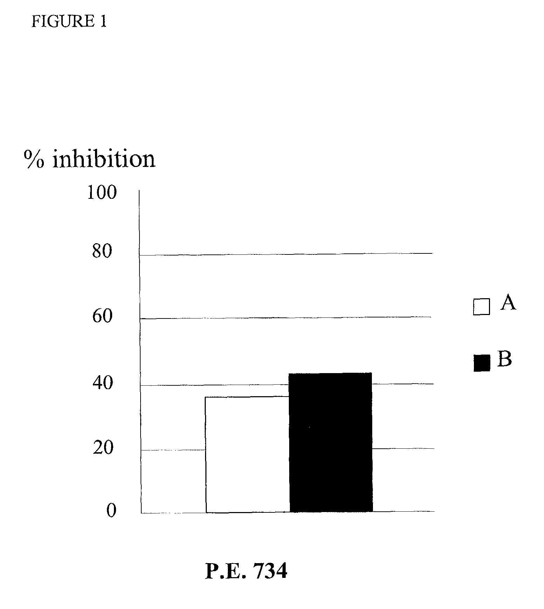

### Figure 1 : comparision of measured inhibition values for the Calvaria Assay (A) and the Pit Assay (B) for the extract of fruits of Amelanchier alnifolia (10 µg/ml) : P.E. 734.

### Examples

### Example 1 : Effect of Amelanchier and Taraxacum species on bone formation

Extracts of the plant species *Amelanchier* and *Taraxacum* were tested for their potential to induce bone formation or inhibit bone resorption.

### Materials and methods

### Preparation of extracts for screening assays:

The ground plant material is defatted with hexane then extracted with a mixture of alcohol and water, with different percentages of water from 10 to 90%, preferably with 50%. The alcohols can be methyl or ethyl alcohols, giving the extract 1a.

On an aliquot of the residue of this first extract, an enzymatic hydrolysis is carried out with a and b glucosidases. Enzymes can be replaced by acidic conditions. The operation may be done under mild conditions (room temperature) or through reflux with different acid concentrations. The aqueous hydrolysed phase is extracted with a non-miscible solvent, preferably ethylacetate to give the extract 2a.

The extract can be dried, freeze-dried or supplied as a liquid form.
In some cases, polyphenols can be discarded through a polyvinylpolypyrrolidone (PVPP) treatment, avoiding artefact with the screening assays.

Following the extract preparation, each extract was weighed, redissolved in dimethylsulphoxide (DMSO) to a final concentration of 20 mg/ml and stored in aliquots at -20°C. This was used as a stock solution and was subsequently diluted in media for each assay. A range of doses was tested in the assay systems.

Extracts of *Amelanchier ovalis* (P.E. 219 (MeOH/water)), and of *Taraxacum officinale* (P.E. 750 (ethylacetate)) were further tested in a human periosteal /preosteoblast cell line, hPOB-tert for their ability to induce the endogenous expression of BMP-2.

The validation of this assay was performed with statins as a positive control. At confluence, cells were incubated with 0.05 mg/ml Lovastatin or with the plant extracts. Total RNA was extracted with TRIzol Reagent (Gibco). 10 µg RNA were reverse transcribed using the 1st Strand cDNA Synthesis Kit (Boehringer). BMP-2 cDNA sequences were amplified for 35 cycles at an annealing temperature of 55°C using specific oligonucleotide primers (5':TTGCGGCTGCTCAGCATGTT; 3':CATCTTGCATCTGTTCTCGGAA). PCR products were separated by agarose gel electrophoresis and detected by ethidium bromide staining. Quantification was performed using NIH Image Software and normalizing results with Actin as housekeeping gene.

### Results :

For *Amelanchier ovalis* : induction of BMP-2 by 2.5 fold.
For *Taraxacum officinale* induction of BMP-2 by 2.0 fold.

### Example 2 : Effect of Amelanchier and Taraxacum species on bone resorption

The ability of the extracts prepared as in example 1, to inhibit IL-1 (10⁻¹⁰ M) stimulated bone resorption was assessed in the neonatal bone resorption assay. Each extract was assessed for its capacity to inhibit bone resorption at 10 µg/ml

Extracts of fruits of *Amelanchier alnifolia* (10 µg/ml) (P.E. 734 (ethylacetate)) were able to inhibit IL-1 induced bone resorption in the murine calvaria assay ( R.J. Murills, "In vitro Bone Resorption Assays" in Principles of Bone Biology (Academic Press) 1986, chap. 90). This effect was confirmed in a second bone resorption test, namely the pit assay using rabbit bone mixed cell cultures on bovine bone slices (Tezuka K., et al., 1992, *Biochem*. *Biophys*. *Res*. *Commun.* **186**(2):911-7 and Lorget F., et al., 2000, *Biochem*. *Biophys*. *Res*. *Commun.* 268(3):899-903) . Resorption pits are visualized by staining for TRAP (tartrate resistant acid phosphatase) positive cells and counted.

A comparison of activity of the extracts at 10 µg/ml in the two assay systems is shown in Figure 1.

### Example 3: Dry pet food

A feed mixture is made up of about 58% by weight of corn, about 5.5% by weight of corn gluten, about 22% by weight of chicken meal, 2.5% dried chicory, about 10% of extract of *Taraxacum* leaves, salts, vitamins and minerals making up the remainder.

The feed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food has a positive effect on bone and cartillage health and increase their mobility.

## Claims

1. A food composition intended for the prevention, alleviation and/or treatment of bone disorders or maintenance of bone health in humans and pets, which comprises as an active ingredient an effective amount of at least one plant or plant extract selected from the group consisting of *Taraxacum* and *Amelanchier*.

2. A composition according to claim 1, wherein the plant or plant extract is selected from the group consisting of *Taraxacum officinale* (common dandelion), *Taraxacum kok-saghyz*, *Amelanchier ovalis*, *Amelanchier alnifolia*, *Amelanchier laevis*, *Amelanchier arborea* and *Amelanchier asiatica*

3. A composition according to claims 1 or 2, wherein the plant or plant extract is from leaves or roots of *Taraxacum* species, or fruits of *Amelanchier species,* or a mixture thereof.

4. A composition according to one of claims 1 to 3, wherein the plant extract is used alone or in association with other plants such as chicory, tea, cocoa, or with other bioactive molecule such as antioxidants, fatty acids, prebiotic fibers, glucosamine or chondroitin sulphate.

5. A composition according to one of claims 1 to 4, which is in the form of a nutritionally balanced food or pet food, a dietary supplement, a treat or a pharmaceutical composition.

6. A composition according to one of claims 1 to 5, which helps bone regenaration during fracture healing, helps to increase bone formation and bone mineral density during growth and optimize peak bone mass or to decrease bone loss, in particular bone loss associated with age in humans or pets.

7. A food composition according to one of claims 1 to 6, which helps to build cartilage in humans or pets.

8. A food composition according to one of claims 1 to 7, which helps to prevent osteoarthritis in humans or pets, which results in a better activity or mobility of the individual.

9. Use of a plant or a plant extract selected from the group consisting of *Taraxacum* or *Amelanchier*, for the preparation of a food or pet food composition intended for the prevention, alleviation and/or the treatment of bone disorders or maintenance of bone health in humans or pets.

10. The use of a plant extract selected from the group consisting of *Taraxacum* or *Amelanchier*, for the preparation of a food or pet food composition intended to help bone regenaration during fracture healing, increase bone formation and bone mineral density during growth and optimize peak bone mass or to decrease bone loss, in particular bone loss associated with age in humans or pets.

11. The use of a plant or a plant extract selected from the group consisting of *Taraxacum* or *Amelanchier*, for the preparation of a food or pet food composition intended for preventing or alleviating the symptoms of osteoartritis in humans or pets, improving activity and/or mobility of the individual.

12. The use of a plant extract selected from the group consisting of *Taraxacum* or *Amelanchier*, for the preparation of a food or pet food composition intended help to build cartilage in humans or pets.

13. The use according to claim 9 or 12, wherein the composition is as described in one of claims 1 to 5.

14. A method of treatment, alleviation or prevention of bone disorders or maintenance of bone health in humans or pets, which comprises administering an effective amount of a composition according to one of claims 1 to 5.

15. A method of increasing bone formation, bone mineral density during growth and optimize peak bone mass in humans or pets, comprising the step of feeding an individual, a composition according to any of claims 1 to 5.

16. A method for the treatment, alleviation and/or prophylaxis of osteoarthritis in humans or pets, comprising the step of feeding the individual, a composition according to any of claims 1 to 5.

17. A method of treating or preventing osteoporosis, comprising administering an effective amount of a composition according to any one of claims 1 to 5.

18. A method of stimulating bone regenaration during fracture healing, comprising the step of feeding an individual, a food composition according to any of claims 1 to 5.

19. A method of decreasing bone loss, in particular bone loss associated with age in humans or pets, comprising the step of feeding the individual, a food composition according to any of claims 1 to 5.
